# EUROPEAN PATENT SPECIFICATION

(11) **EP 2 315 605 B1**
(45) Date of publication and mention of the grant of the patent: **28.11.2012**
(21) Application number: 09777639.7
(22) Date of filing: 04.08.2009
(51) Int. Cl.: A61L 15/28, A61L 15/32, A61L 15/44, A61L 24/00, A61L 15/20, A61L 24/08, A61L 24/10, A61L 27/20, A61L 27/24, A61L 27/54

(54) **CHOLESTERYL SULFATE-CONTAINING COMPOSITION AS A HAEMOSTATIC**
CHOLESTERYLSULFAT ENTHALTENDE ZUSAMMENSETZUNG ALS HÄMOSTATIKUM
COMPOSITION CONTENANT DU SULFATE DE CHOLESTÉRYLE EN TANT QU'AGENT HÉMOSTATIQUE

(30) Priority: 04.08.2008 US 85900 P; 11.02.2009 EP 09152551
(43) Date of publication of application: 04.05.2011
(73) Proprietor: Dr. Suwelack Skin & Health Care AG, 48727 Billerbeck (DE)
(72) Inventor: WIELAND, Martin, 48653 Coesfeld (DE); HAYWARD, James A., Stony Brook New York 11790 (US)
(74) Representative: Gille Hrabal
(86) International application number: PCT/EP2009/005636
(87) International publication number: WO 2010/015378

(56) References cited:
- EP-A1- 1 695 722
- WO-A-01/24839
- WO-A-91/01719
- WO-A-91/08745
- WO-A2-2006/009987
- WO-A2-2007/127236
- SHIMADA T ET AL: "Activation of factor XII and prekallikrein with cholesterol sulfate" THROMBOSIS RESEARCH, TARRYTOWN, NY, US, vol. 38, no. 1, 1 April 1985 (1985-04-01), pages 21-31, XP022881572 ISSN: 0049-3848 [retrieved on 1985-04-01]
- DATABASE WPI Week 19773 Thomson Scientific, London, GB; AN 1977-04726Y XP002534326 & JP 51 139634 A (TAMIYA K) 1 December 1976 (1976-12-01) cited in the application
- DATABASE WPI Week 19998 Thomson Scientific, London, GB; AN 1999-090062 XP002534327 & JP 10 324633 A (SEIKAGAKU KOGYO CO LTD) 8 December 1998 (1998-12-08) cited in the application
- HIDEKATSU YANAI ET AL.: "EXPRESSION OF CHOLESTEROL SULFOTRANSFERASE (SULT2B1b) IN HUMAN PLATELETS" CIRCULATION, 2004, pages 92-96, XP002534324 cited in the application
- MAMORU KYOGASHIMA ET AL.: "ROLES OF GALACTOSE AND SULFATE RESIDUES IN SULFATIDES FOR THEIR ANTAGONISTIC FUNCTIONS IN THE BLOOD COAGULATION SYSTEM" GLYCONJUGATE JOURNAL, 2001, pages 245-251, XP002534325 cited in the application
- KYOGASHIMA M: "The role of sulfatide in thrombogenesis and haemostasis" ARCHIVES OF BIOCHEMISTRY AND BIOPHYSICS, ACADEMIC PRESS, US, vol. 426, no. 2, 15 June 2004 (2004-06-15) , pages 157-162, XP004509792 ISSN: 0003-9861

## Description

The present invention relates to a composition comprising a wound dressing device and cholesteryl sulfate and in particular to a composition containing cholesteryl sulfate and at least one carrier material for use as a haemostatic agent, as well as to a method for its preparation and use.

Cholesteryl sulfate, also known as cholesterol sulfate, is a natural constituent of the human body. The plasma level of cholesteryl sulfate is within the range of 150 to 350 µg /100 ml. Cholesteryl sulfate is also a constituent of cells and membranes. In the body, cholesteryl sulfate assumes numerous regulative functions; inter alia, it affects serin protease activity. As a constituent of membranes, cholesteryl sulfate stabilizes erythrocytes against osmotic fluctuation and supports thrombocyte adhesion.

In addition, it plays an important accelerating role in the differentiation and maturation of keratinocytes and, thus, has a strong influence on the regulation of the skin barrier function (Strott, 2003; Merten, 2001).

With respect to supporting the skin barrier function, numerous examples for relevant cosmetic applications are known from the prior art. For instance, the patent families of WO 00/45786, WO 01/74327 and WO 02/060381 disclose cholesteryl sulfate-containing compositions for the topical application of dry skin, in particular for enhancing the lipid barrier function of the skin or the barrier function of the stratum corneum. Documents JP 11005742, JP 5051314 and JP 60161911 also disclose the application of cholesteryl sulfate in skin care. Other cosmetic applications of cholesteryl sulfate, for example for hair and nail care, are found in the documents JP 01305018, JP 1305014 and JP 3145412.

This prior art does not yield any suggestions that further positive active properties, in particular in the area of haemostasis, are to be expected beyond the action of cholesteryl sulfate in the stratum corneum.

Moreover, lipid compounds are known which contain cholesteryl sulfate in the form of lipidic globules or in liposomes respectively. Such lipid vesicles and compositions containing such lipidic cholesteryl sulfate compounds as well as their use in cosmetic preparations are, for example, the subject matter of the patents DE 69006811, DE 69203623, DE 69809139, DE 69900264 and DE 69904011.

From WO 91/01719 liposomes consisting of bovine brain ceramide, cholesterol, palmitic acid and cholesteryl sulfate for entrapping small peptides as active ingredient against skin lesions induced by virus, bacteria, inflammation or other causes by treatment of infected epithelial cells are known.

The disclosure of these documents also does not permit any conclusions as to any haemostatic activity or blood-coagulatory promoting properties of cholesteryl sulfate or of compositions containing it.

From the area of pharmaceutical applications of cholesteryl sulfate, the administration for treating mycoses can be found in the patent family of WO 88/06450 as well as in US 5,194,266, with pharmaceutical compositions from amphotericin B and cholesteryl sulfate particles being disclosed in this context. The composition mentioned therein is applied parenterally or intramuscularly preferably in liquid form. A composition comprising cholesteryl sulfate and a carrier for use as a haemostatic or a composition in the form of a layer is not disclosed. The treatment of mycoses using cholesteryl sulfate-containing compositions differs significantly from the effect upon blood clotting.

WO 91/08745 refers to compositions containing cholesterol-3-sulfate and a pharmaceutically acceptable carrier for use in the prevention and treatment of rotavirus infections. Any effects of cholesteryl sulfate on blood clotting are not subject of the disclosure. As pharmaceutically acceptable carrier usual excipients such as mannitol, lactose, starch, magnesium stearate etc. are mentioned. The composition is indicated for oral application in the form of solutions, tablets, pills or powders. Compositions which are not indicated for oral application and/or are present as layers are not disclosed.

From JP 51139634 the use of cholesteryl sulfate in compositions for the regulation of blood pressure is known. The compositions are mixtures of the ingredients in a solvent namely as liquids or emulsions. Compositions comprising cholesteryl sulfate and a carrier for use as a haemostatic and/or a compositions being present as a layer are not disclosed.

JP 51139634 mentions no indications that cholesteryl sulfate has a positive effect on haemostasis beyond the actions disclosed and/or that cholesteryl sulfate is combined with a carrier to form a composition in the form of a layer.

Investigations as to the influence of cholesteryl sulfate both on the plasmatic coagulation system and the blood platelets are described in literature. It could be demonstrated that a strong triggering of the intrinsic coagulation system is effected by cholesteryl sulfate through the activation of Factor XII and prekallikrein (Shimada, 1985). Accordingly, cholesteryl sulfate is capable of accelerating aggregation of blood platelets.

If thrombocytes are being activated by cholesteryl sulfate, they exhibit a strong degree of agglomeration. Obviously, such an effect cannot be caused by the use of similar molecules but appears to be very specifically limited to cholesteryl sulfate (Merten, 2001; Blache 1995).

However, there are also descriptions of cholesteryl sulfate having an inhibiting effect on thrombin, but thrombin must first be incubated with cholesteryl sulfate in order to generate this effect (Iwamori, 1999). The investigations of the mode of action of cholesteryl sulfate described herein are limited to selected regulation systems and very specific partial aspects of the coagulation cascade, and in each case only form parts of the highly complex and comprehensive interplay of highly specific individual reactions, even with contradictory results having been obtained in part.

Instead Hidekatsu Yanai et al. (2004) generally refers to the ability of cholesterol sulfate to influence blood clotting and fibrinolysis and its effect on human platelets where it has been shown to support platelet adhesion. Nevertheless this document does neither refer to a composition comprising cholesteryl sulfate and carrier material for use as a haemostatic agent nor does it disclose a composition in the form of a layer containing cholesteryl sulfate and a carrier material.

The same holds true for the investigations of Mamoru Kyogashima (2007) wherein cholesterol-3-sulfate was administered intravenously dissolved in a buffer solution and its coagulant activity was examined. Only found was a general haemostatic action of cholesteryl sulfate but no disclosure of a combination with a carrier material for haemostatic use and/ or for a composition in the form of a layer comprising cholesteryl sulfate and a carrier can be found herein.

From JP 10324633 topical blood coagulation promoters for use as a haemostatic agent are known. The blood coagulation promoter is cholesterol sulphur. This document does neither disclose compositions comprising cholesteryl sulfate and/or a carrier for use as a haemostatic nor a composition in the form of a layer.

According to the present invention it was now found that a composition containing cholesteryl sulfate in or on at least one carrier material shows particular suitability for serving as an effective haemostyptic device. In addition, the combination of cholesteryl sulfate with a carrier material makes an optimal form of application possible. By suitably selecting the carrier material, e.g. collagen, alginate or chitosan, the haemostatic properties could possibly be further positively enhanced. The combination of cholesteryl sulfate with a carrier material for the application as a haemostyptic cannot be found as having been previously published in the prior art.

Known haemostyptics are based on compositions containing substances or activators of the coagulation cascade, such as, e.g., thrombin or fibrinogen. Thus, the subject matter of WO 03/094983 is thrombin-releasing spheres. Haemostatic collagen sponges with thrombin or thrombin precursors are known from EP 0891193 or US 4,948,540. EP 0485210 and EP 1053756, for example, disclose combinations of carrier materials with fibrinogen as a haemostatic.

A drawback of these haemostatic systems is, on the one hand, their high degree of susceptibility and low stability of the haemostatic active agents thrombin and fibrinogen vis-à-vis external influences, such as humidity, pH value and temperature. Moreover, these active agents are based on materials of human or animal origin and are thus potentially carriers of pathogenic substances, such as viruses that could originate from the source material. Moreover, obtaining and processing these active agents is very complex and cost-intensive, also due to the high risk of infection.

The haemostatic effect of certain carrier materials, such as sponges of pure collagen or chitosan, is also known from the prior art, as US 6,454,787 and WO 2005/062896, for example, show.

WO2007/127236 discloses an article of manufacture for use with a percutaneous device, comprising a matrix and an antimicrobial agent. The matrix may comprise a hydrophilic matrix material such as collagen, hyaluronic acid, alginate... The matrix may comprise further additives such as cholesteryl sulfate.

WO2006/00987 discloses a composition comprising an effective amount of a labile agent incorporated into a water-soluble polymeric film and an additive composition. The labile active may be cholesterol sulfate.

EP1695722 discloses a collagen haemostatic foam comprising other haemostatic agents and therapeutic agents.

Such pure haemostatic carrier materials based on pure collagen or chitosan exhibit a comparatively low haemostatic performance. In particular, compared to highly potent active agents such as thrombin or fibrinogen, or with systems additionally doped with active agents, the pure carrier materials are inferior as regards their haemostatic performance. However, a fast and effective coagulation effect is advantageous in particular in the case of wounds that bleed heavily.

Based on the prior art, the object to be solved by the present invention lay in providing a composition that is suitable for enabling effective haemostasis with a good application facility, high degree of safety and good availability with respect to the raw materials used. The inventors of the present invention found that the above-described problems of the prior art could be solved by the combination of cholesteryl sulfate with suitable carrier materials.

Thus, the invention provides an effective haemostatic that offers good applicability and effectiveness, and which is significantly more cost-effective and entails less risk than conventional agents in its manufacture.

WO 01/24839 refers to a pharmaceutical composition for use as a wound dressing device for the treatment of chronic and especially infected dermal wounds. One object of the invention is a wound dressing device comprising a cross-linked polymer and a non-gellable polysaccharide matrix which additionally comprises a water loss control agent which may be selected from cholesteryl sulfate. The cholesteryl sulfate therefore exhibits specific physico-chemical properties (water loss control) which are necessary in the very specific wound dressing matrix of cross-linked polymer and non-gellable polysaccharide and which are furthermore essential for the very specific application of such wound dressing matrix in the field of treatment of chronic and infected wounds. The document does therefore not refer to compositions in the form of a layer containing a carrier and cholesteryl sulfate as a pharmaceutically active ingredient. Carrier which consists only of one natural or synthetic polymer or carrier in particular selected from freeze-dried collagen matrices, freeze-dried alginate matrices, freeze-dried hyaluronic acid matrices, freeze-dried chitosan matrices, freeze-dried collagen-alginate matrices, freeze-dried alginate-hyaluronic acid matrices, freeze-dried alginate-polyacrylic acid matrices or woven fleeces, compresses or gauzes in combination with cholesteryl sulfate are not disclosed in WO 01/24839 either.

Accordingly, subject of the present invention are compositions comprising cholesteryl sulfate and at least one carrier material for use as a haemostatic agent (sometimes also referred to as haemostatic), compositions which are present as a layer, comprising cholesteryl sulfate and a carrier material, wherein a carrier material comprising a mixture of a cross-linked polymer and a non-gellable polysaccharide is excluded, as well as methods for the preparation of such compositions and their use.

Cholesteryl sulfate, also referred to as cholesterol sulfate or 5-cholestene-3-β-ol-sulfate or cholesterol-3-suffat, respectively, is a derivative of cholesterol with the characteristic steroid skeleton and a sulfate group. 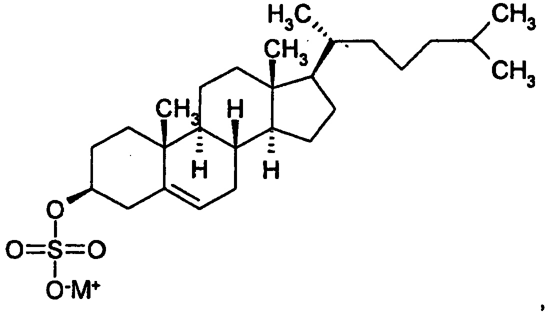 wherein M is selected from the group consisting of alkaline or alkaline earth metals (in the latter divalent metals M naturally corresponds to half an equivalent), or where M⁺ is an ammonium cation, as NH₄⁺ or a mono, di, tri or tetraorganoammonium. Peferably, M is an alkaline metal, such as, in particular, sodium or potassium.

Particularly preferably, cholesteryl sulfate is present as a potassium or sodium salt. Compared with sodium cholesteryl sulfate, potassium cholesteryl sulfate is characterized by greater stability, but is less soluble, e.g. in methanol. Sodium cholesteryl sulfate is most preferably used, according to the invention, particularly as sodium is physiologically safer than potassium. Both sodium cholesteryl sulfate as well as potassium cholesteryl sulfate are commercially available.

The carrier material preferably is a hydrophilic material, i.e. a material that is wettable with water. Preferably, it is a so-called hydrocolloid, that is a partly water-soluble or water-swellable natural or synthetic polymer. Particularly preferred are hydrocolloids from the groups of proteins, polysaccharides, glucosaminoglycanes and/or synthetic polymers and foams.

Preferably, the carrier material is selected from the group of proteins, such as e.g. collagen, gelatin, elastin, keratin, fibroin, albumin, globulins such as lactoglobulin, milk proteins such as casein, etc., or mixtures thereof, with collagen being particularly preferred. Collagen-based carrier materials are preferably such materials as are processed and prepared according to methods known from the prior art and, for example, from DE 4028622. The collagen carrier materials preferred according to the invention are characterized, in particular, by excellent hydration properties and good absorbency, an aspect which is advantageous in particular with regard to the absorption of large quantities of liquid, for example in the case of heavily bleeding wounds. Due to the structural similarity with human skin and human tissue, collagen types occurring in skin and tissue are particularly selected, in particular collagen of the types I, III and V. This causes the particularly good compatibility and biocompatibility of such collagen carrier materials according to the invention. Moreover, the agents obtainable in this manner are biodegradable in the body and can be metabolized in a natural manner when remaining in a wound. Thus, such carrier materials are particularly suitable for the preparation of haemostatics for use as an implant. The collagen carrier material used according to the invention is preferably obtained from collagen sources of bovine, equine and porcine origin. Very particularly preferred is bovine collagen. The collagen can be obtained according to usual methods from the usual sources, such as skins or sinews. Mixtures of, for example, collagen and gelatine or particularly preferably of collagen and elastin can also be used. Furthermore, collagen materials that have been subjected to a cross-linking treatment can be used according to the invention. In this case, a thermal cross-linking, the so-called dehydrothermal cross-linking, is preferred, or also cross-linking with chemical cross-linking agents, such e.g. with aldehydes, such as glutaraldehyde, carbodiimides, such as EDC, isocyanates, epoxides or imidazoles, with epoxide being particularly preferred from the group of chemical cross-linking agents.

Carrier materials from the group of polysaccharides include, for example, homoglycanes or heteroglycanes, such as, for instance, alginates, in particular sodium alginate, carrageen, pectins, gum tragacanth, guar gum, carob gum, agar-agar, gum arabic, xanthan gum, natural and modified starches, dextrans, dextrin, maltodextrin, chitosan, glucans, such as ß-1,3-glucan or ß-1,4-glucan, cellulose etc. Particularly preferred polysaccharides are alginates, in particular sodium alginate and calcium alginate.

Glucosaminoglycane (mucopolysaccharides) include, for example: hyaluronic acid, chondroitin sulfate, dermatan sulfate, keratan sulfate, heparan sulfate, heparin, etc. Hyaluronic acid is particularly preferred.

The group of synthetic polymers comprises, for example: cellulose ether, polyvinyl alcohol, polyvinyl pyrrolidone, synthetic cellulose derivatives, such as methylcellulose, carboxycellulose, carboxymethylcellulose such as, for instance, sodium carboxymethylcellulose, cellulose ester, cellulose ether such as hydroxypropylcellulose, polyacrylic acid, polymethacrylic acid, poly(methylmethacrylate) (PMMA), polymethacrylate (PMA), polyethylene glycols, polyurethanes, polyurea compounds etc. Particularly preferred are sponges and foams of acrylates.

Mixtures of several carrier materials can also be used. In this case, particularly preferred are mixtures, e.g. of collagen and alginates, such as, e.g., calcium alginate, or of alginates and hyaluronic acid, or of alginates and polyacrylate or polyacrylic acid, and mixtures having more than two carrier material components can also be constituents of the compositions according to the invention.

In preferred embodiments according to the present invention the carrier is selected from freeze-dried collagen matrices, freeze-dried alginate matrices, freeze-dried hyaluronic acid matrices, freeze-dried chitosan matrices, freeze-dried collagen-alginate matrices, freeze-dried alginate-hyaluronic acid matrices, freeze-dried alginate-polyacrylic acid matrices. Preferably such freeze-dried matrices are present in the form of sheets, layer, pads, films or foams.

Carrier materials from the group of conventional wound dressings are also preferred. Such conventional wound dressings are common water-wettable dressing materials or wound dressings, in particular those based on woven or fibre-containing fleeces, compresses or gauzes of cotton, mull, cellulose, viscose, staple fibre, acrylic fibres, polyester, polyethylene, polyamide, polyurethane, polyurea compounds or mixtures thereof. Examples of such conventional wound dressings which can be used as carrier materials can be found, for example, in "Wundauflagen für die Kitteltasche" - A. Vasel-Biergans, Wissenschaftliche Verlagsgesellschaft mbH Stuttgart, 2nd edition, 2006.

One aspect of the present invention is a composition which is present as a layer, comprising cholesteryl sulfate and a carrier material, wherein a carrier material of a mixture of a cross-linked polymer and a non-gellable polysaccharide is excluded.

Non-gellable polysaccharides comprise non-gellable galactomannan macromolecules such as guar gum, lucerne, fenugreek, honey locust bean gum, white clover bean gum and carob locust bean gum.

A further aspect of the present invention is a composition wherein the carrier material is selected from those comprising only one of the above mentioned carrier materials and does not constitute a mixture. Preferably such embodiments comprise only one carrier material selected from the group of natural or synthetic polymers. Most preferred are such embodiments wherein the carrier material is selected from collagen or chitosan or alginate. Preferably the collagen, Chitosan or alginate carrier is in the form of a freeze-dried matrix, which are particularly present in the form of sheets, layer, pads, films or foams.

Nevertheless such embodiments comprising only one carrier material can additionally contain, besides cholesteryl sulfate which itself can be classified as a therapeutical and/or pharmaceutical active agent, one or more further active agents and/or one or more auxiliary substances.

Still another aspect of the present invention are compositions wherein the carrier material is selected from freeze-dried collagen matrices, freeze-dried alginate matrices, freeze-dried hyaluronic acid matrices, freeze-dried chitosan matrices, freeze-dried collagen-alginate matrices, freeze-dried alginate-hyaluronic acid matrices, freeze-dried alginate-polyacrylic acid matrices or woven fleeces, compresses or gauzes. Preferably such carrier materials are present in the form of sheets, layer, pads, films or foams.

Blood coagulation is a system first requiring an impulse for the initiation of blood coagulation with a row of enzymatic steps being subsequently activated which finally lead to the formation of a clot. The activation of the extrinsic pathway by the tissue factor (thromboplasmine) is generally considered the trigger of plasmatic coagulation. Alternative steps include the activation of thrombocytes as well as a stimulus via factor XII, the actual extent of the influence of this mechanism upon blood coagulation in vivo being the subject of controversial debate.

Once blood coagulation has been initiated, it processes in a cascade-like manner until a clot is formed, the catalytic effect of the enzymes generally causing exponential rather than linear steps. In an intact coagulation system, coagulation generally is self-supporting, and other stimulants only have a limited effect on it.

Nevertheless the inventors surprisingly found that compositions comprising cholesterol sulfate as a haemostatic agent and a carrier material with haemostatic activity, however, exhibited a synergistic or enhanced coagulation effect. Which means the combination of cholesteryl sulphate as an haemostatic agent with a haemostatic carrier material exhibits an increased effect in blood clotting compared to cholesteryl sulphate or the carrier material alone.

Therefore according to the invention, carrier materials are preferred that as such already exhibit haemostatic effects, such as, for example, collagen, alginate or chitosan, with collagen and chitosan being particularly preferred.

The composition according to the invention can contain, besides cholesteryl sulfate which itself can be classified as a therapeutical and/or pharmaceutical active agent, one or more additional therapeutic and/or pharmaceutical active agents. These are active agents, which, within the meaning of the Drugs Act are intended, among other things, to heal, alleviate or prevent diseases, discomfort, bodily defects or pathological complaints. Such additional active agents can be substances which are also haemostatically effective, such as, for example, thrombin and fibrinogen. Furthermore, agents having an activating effect upon factors and substances of the extrinsic and/or intrinsic coagulation cascade can be used as further active agents, such as, for example, phospholipids, kaolin, aprotinin, factors or factor concentrates, tissue factor or calcium ions. Use of active agents having other effects such as, for example, antiseptic, antibacterial, antimycotic, antiparasitic, antiviral, analgetic, antiphlogistic, anaesthetic, immunosuppressive or other beneficial active properties is also possible.

The composition according to the invention further optionally contains one or more auxiliary substances. Auxiliary substance include, for example: fatty substances such as mineral oils, paraffin oils or vaseline oils, silicon oils, refined or unrefined vegetable oils, vegetable lecithins (e.g. soy lecithin), sphingolipids/ceramids isolated from plants, animal oils or fats, fatty acid esters, esters of fatty alcohols and waxes having a melting point corresponding to skin temperature (animal waxes, mineral waxes and synthetic waxes), as well as all oils suitable for cosmetic and medical purposes, such as mentioned in the CTFA treatise, Cosmetic Ingredient Handbook, 1st ed., 1988, The Cosmetic, Toiletry and Fragrance Association, Inc., Washington, polyunsaturated fatty acids, essential fatty acids, surface-active agents such as dispersing agents, emulgators, etc. fillers, pH-regulating agents, such as buffering substances, stabilizers, cosolvents, pharmaceutically and cosmetically common or other colorants and pigments, preservatives, softening agents, lubricants or slip additive agents, etc. Particularly preferred auxiliary substances are selected from the group of pH-regulators, with particularly preferred auxiliary agents being buffers, such as, for example, HEPES or phosphate buffers, or also volatile acids, such as lactic acid or acetic acid.

The classification of the above-mentioned substances into the category of auxiliary substances within the context of the present invention does not preclude these auxiliary substances from also having certain therapeutic effects.

The composition according to the invention is preferably present as a sheet, layer, fleece, film, compress or plaster or foams. Foams are obtainable by providing the carrier material in thick formats. Compositions in the form of layers, matrices, sheets, foams or pads are particularly preferred.

A particularly preferred composition according to the invention has one, several or all of the following features:
- it contains at least 50% by wt of one or more carrier materials, preferably from the group of natural hydrocolloids, particularly preferably collagen,
- it contains at least 0.03 % by wt of cholesteryl sulfate, preferably sodium cholesteryl sulfate,
- it contains 0 to 40% by wt of one or more further active agents
- it contains 0 to 40% by wt of one or more auxiliary substances,
- it preferably contains less than 25% by wt, more preferably less than 20% by wt, still more preferably less than 10% by wt of water,
with the weight specification in each case being relative to the total composition.

In addition, the composition according to the invention, as, for example, the above-mentioned composition containing at least one carrier material, cholesteryl sulfate, preferably sodium cholesteryl sulfate as well as optionally one or more additional active agents, as well as optionally one or more auxiliary substances, preferably has at least one of the following features:
- the geometric form of a sheet, a fleece, a layer, a dressing, a compress or a plaster,
- a thickness (shortest distance of two points, i.e. layer thickness) of 0.04 mm to 50 mm, preferably 0.1 mm to 20 mm, still more preferably 0.4 mm to 10 mm,
- a surface area (surface between two longest side lengths) of 0.25 cm² to 1200 cm², preferably 0.5 cm² to 500 cm², still more preferably 1 cm² to 200 cm².

The cholesteryl sulfate, preferably the sodium cholesteryl sulfate, is present in the composition in a quantity of at least 0.03 % by wt relative to the total composition, and can preferably be worked in up to a quantity of approximately 25% by wt. Here, the cholesteryl sulfate is present in the carrier material in a homogeneous distribution, or is applied onto the carrier material onto the surface of the carrier material, for example as a coating or an impregnation.

In this case, homogeneous distribution is understood to mean an equal distribution, within small statistical fluctuation ranges, over and throughout the entire composition.

To this end, the cholesteryl sulfate can be introduced by admixing into a solution, suspension or mass of the carrier material, preferably into a collagen mass, in the carrier material, with the cholesteryl sulfate being dissolved in a suitable solvent, dispersed or slurried or brought onto suitable carrier particles, prior to admixing.

In a preferred embodiment, the cholesteryl sulfate is worked in in the form of an aqueous vesicle solution.

In one aspect of the invention it is preferred to provide compositions comprising cholesteryl sulfate and a carrier material wherein the cholesteryl sulfate is, in particular, homogeneously, distributed throughout the composition. Such compositions are suitable for cosmetic, medical or pharmaceutical use.

In another preferred embodiment, the cholesteryl sulfate is brought onto suitable carriers particles, preferably made of gelatine. However, other suitable particles can also be used, for example based on natural polymers, such as, for example, polysaccharides, in particular alginates, or particles of glass or plastics. There is, in particular, the option of selecting the carrier particles from a biocompatible and/or a material that is structurally or chemically similar to the carrier material into which the particles are introduced after coating them with the cholesteryl sulfate.

Furthermore, compositions in which the cholesteryl sulfate is applied on suitable carrier materials are also the subject matter of the present invention. For this purpose, the carrier materials can be impregnated, on the one hand, by dipping, steeping or by dripping a solution of the cholesteryl sulfate or a salt thereof, and optionally subsequent drying.

Furthermore, however, an application by coating is also possible. When coating the surface of the carrier material, a concentration gradient of cholesteryl sulfate from the surface of the carrier material to the points of the carrier material distant from the surface is to be expected. "Surface of the carrier material" denotes all exterior surfaces of the composition. In the process, the cholesteryl sulfate is applied onto at least one exterior surface or at least a part thereof. Furthermore, the carrier material can also be coated with the cholesteryl sulfate on several selected or all exterior surfaces, or on several different parts thereof. Preferably, coating is performed by spraying the cholesteryl sulfate onto the carrier material, so called spray coating. For this purpose, the so-called airbrush technique is particularly preferred. Alcoholic solutions, particularly preferably methanolic solutions of cholesteryl sulfate, are sprayed onto a suitable carrier material and optionally dried for spraying or spray coating.

However, it is also possible to apply the coating by printing techniques such as screen printing, pad printing or plane squeegeeing a solution of the cholesteryl sulfate or a salt thereof, and optionally subsequent drying the carrier materials thus coated or impregnated.

The step of drying, which optionally follows the application of the cholesteryl sulfate, can be carried out by conventional drying techniques, with a step of freeze-drying being particularly preferred.

The composition according to the invention is preferably obtainable by a method comprising the following steps:
1. preparing an aqueous solution or suspension of at least one carrier material,
2. admixing of cholesteryl sulfate and, optionally, one or more active agents and/or auxiliary substances,
3. pouring the mixture from step 2,
4. drying the mixture.

In this case, the pouring from step 3 can be carried out into moulds or by methods known from the manufacture of conventional dressing and plaster materials. Pouring the mixture onto a further carrier material is also possible, such as, for example, a synthetic film, a plaster cover with an adhesive layer, or the like.

A method is particularly preferably used wherein the pouring of the mixture from step 2 is carried out into a mould, followed by freezing the mixture in the mould, and wherein the drying from step 4 is done by freeze-drying the frozen mixture while forming a moulded article. The moulded article thus obtained can optionally be further adjusted by cutting or laminating.

Further steps could optionally be carried out between this steps, in particular, it is possible to adjust the pH value of the solution or suspension in step such that an optimum homogenization and admixing or, optionally, also stabilization of the selected further active agents or auxiliary substances is possible. Optionally, the mass can be subjected to further mechanical processing for the purpose of homogenization following step 2.

Expediently, the manufacturing procedure is performed by first preparing an aqueous solution or suspension of the carrier materials in step 1, and then adding and mixing the desired further active agents and auxiliary substances. Optionally, processing can be carried out dependent on temperature and/or pH value, in accordance with the type and stability of the active agents and auxiliary substances used.

Then, in step 2, cholesteryl sulfate is added by mixing in cholesteryl sulfate, preferably sodium cholesteryl sulfate.

In a preferred variant of the method, cholesteryl sulfate, in particular sodium cholesteryl sulfate, is mixed in in the form of an aqueous vesicle solution of cholesteryl sulfate. Such a vesicle solution can be prepared by suspending cholesteryl sulfate in water and treating this suspension with ultrasound. In the process, micelle-like vesicles with a positive charge distribution in the interior of the vesicles and a negative charge distribution on the exterior of the vesicles form. In order to prepare such aqueous vesicle solutions, quantities of 0.5 to 4 % by wt sodium or potassium cholesteryl sulfate are subjected to sound waves at temperatures ≧ 80°C, more preferably ≧ 85°C, still more preferably ≧ 90°C at 10 - 75 watts, preferably at 50 - 70 watts, for example in an ultrasonic device having a sonotrode, for example with a Sonoplus-device by the company Bandelin. Both K- as well as Na-cholesteryl sulfate are equally suitable for preparing the vesicle solution. The vesicle solution is then mixed into the solution or suspension of the carrier material, preferably into a collagen mass. The advantage of such a cholesteryl sulfate vesicle solution over a conventional aqueous solution lies in the fact that higher concentrations of the cholesteryl sulfate can be transferred into a stable solution or suspension. Both the potassium cholesteryl sulfate preferred according to the invention, and the particularly preferred sodium cholesteryl sulfate are characterised by an only small water-solubility, so that only a very limited proportion of the effective cholesteryl sulfate is available in the aqueous solution. In contrast, higher and, according to the invention, sufficiently high amounts of active agents can be provided in an aqueous medium by the preparation of cholesteryl sulfate vesicles in an aqueous medium. The solubility of potassium and sodium cholesteryl sulfate in alcoholic solvents, such as, for example, methanol, may be greater than that in water, but the use of such alcoholic solvents, such as, for example, methanol, is disadvantageous due to the toxicological potential. Moreover, aqueous media in the production method preferably used according to the invention are significantly simpler, more harmless and convenient with regard to handling. In particular in a method comprising a freeze-drying process as a drying step, the use of solvent-containing or alcoholic media is not preferred. By using an aqueous cholesteryl sulfate vesicle solution prepared according to the invention, these drawbacks concerning the solubility and toxicity potential or the deficient technical usability can be avoided.

In another preferred variant of the method, the mixing-in is carried out by adding carrier particles coated with cholesteryl sulfate. When using collagen as the carrier material, an alcoholic, preferably methanolic solution of cholesteryl sulfate, preferably in the form of the sodium salt, is preferably added to gelatine particles in a mass ratio of gelatine to cholesteryl sulfate of up to 2:1. The solvent, such as, for example, methanol, is removed by vaporizing, and the cholesteryl sulfate is brought onto the gelatine particles. The cholesteryl sulfate can also be brought onto other suitable carrier particles in an analogous manner. A quantity of 0.0016% by wt to 0.16% by wt of cholesteryl sulfate, which was brought onto the carrier particles, is added to the suspension or solution of the carrier material, with the weight specifications relating to the total quantity of solution or suspension with the coated carrier particles.

If hydrocolloids on a vegetable polysaccharide basis are used as the carrier material of the composition according to the invention, then carrier particles of a structurally related material are selected, e.g., also from the group of polysaccharides, such as, e.g., from the alginates.

In step 3, the solution or suspension thus obtained, which contains at least one carrier material, cholesteryl sulfate, as well as optionally further additional active agents and/or auxiliary substances is casted. This may also comprise, for example, application onto a suitable further carrier material. Preferably, the solution or suspension is casted into a mould in step 3.

Direct drying, by suitable and known drying methods, or, in a particularly preferred embodiment, by freezing the solution or suspension from step 3 in the mould and freeze-drying, follows in step 4.

The solution or suspension may actually cool off or freeze in the mould in an arbitrary manner. Preferably, the cooling-off in the preferably used method according to the invention is effected by cooling plates. Other methods include, for example, blowing with cold air or immersing the moulds into liquid gases, such as immersion into liquid nitrogen. The cooling rate in the process has an effect upon the size of the ice crystals formed. They in turn have an effect upon the pore size distribution of the moulded article formed. If few large crystals are formed, then the moulded article has few large pores. If many small crystals are formed, then the moulded article has many small pores. The higher the cooling off rate of the solution or suspension, the smaller the crystals become.

The freezing temperature required depends, among other things, on how far the freezing point has been lowered due to the active agents or auxiliary substances contained in the solution or suspension. Expediently, the temperature is below the freezing point of water down to the temperature of liquid nitrogen (- 196°C). Preferably, the freezing temperature is about - 20°C to - 80°C.

In a preferred embodiment of the method, the mass is frozen and subjected to freeze-drying in step 4. This can take place in a manner known per se, such as described, for example, in DE 4328329 C2 or DE 4028622 C2. As regards the process parameters, a drying temperature in the range from - 20 to + 100°C in a vacuum of about 10-30 Pa (0.1 to 0.3 mbar) is preferably selected. The freeze-drying process is preferably carried out over a period of time of about 15 to 90 hours. Following the freeze-drying process, the composition according to the invention usually has a residual water content of less than 10%, more preferably less than 5%, still more preferably less than 1%. The freeze dried, generally porous moulded compositions can be subjected to a further subsequent treatment, such as laminating, cutting, punching or stamping or the like.

In order for the freeze-dried composition to have a sufficient mechanical stability it is in general necessary for the solution or suspension to have a certain concentration of the carrier material. Of course, this concentration depends on the type of hydrocolloid. Expediently, it is about at least 0.2% by wt relative to the total quantity of the solution or suspension, preferably 0.4% by wt to 8.0% by wt, still more preferably about 1.0% by wt to 4.0% by wt (weight of the carrier material relative to the total weight of the solution or suspension). Higher or lower concentrations are not preferred, because in that case, the viscosity of the solution or suspension is too low or too high, thus making the solution or suspension more difficult to process. The quantity of the carrier material contained in the solution or suspension decisively affects the density of the composition obtained (weight of the composition relative to the volume of the geometric shape of the composition). The density in turn is an important quantity for the absorbency or liquid absorption capacity of the composition.

The higher the concentration of the carrier material in the solution or suspension, the higher the density becomes, and the lower the degree of porosity of the composition and vice versa. In view of the density/degree of porosity or absorbency/liquid absorption capacity, the concentration of the carrier material in the solution or suspension prepared in step 1 is preferably selected from a range of 1.0% by wt to 3.0% by wt, relative to the solution or suspension. The concentration of the preferably used proteinogen hydrocolloid collagen preferably is from 0.5 to 5, more preferably from 1 to 3% by wt, relative to the solution or suspension.

If polysaccharide-based hydrocolloid, such as, for example, alginate, in particular Na-alginate, is used as carrier material, this is preferably present in an amount from 0.5% by wt to 5.0% by wt relative to the solution or suspension.

Expediently, the densities of the compositions obtained in accordance with this method according to the invention are about 0.005 g/cm³ to 1.0 g/cm³, preferably about 0.01 g/cm³ to 0.5 g/cm³, preferably about 0.02 g/cm³ to 0.2 g/cm³. The term density as it is presently used, denotes the weight of the composition relative to the volume of the exterior geometric shape of the composition.

The composition according to the invention preferably have a pore size distribution of about 10 to 150 µm, more preferably of about 20 to 110 µm.

The absorbency or liquid absorption capacity of the compositions obtained in accordance with the method according to the invention denotes the capability for absorbing liquid quantities, in particular combined with the capability of storing and retaining these absorbed liquid quantities. According to the invention, such compositions are preferred as are capable of absorbing and storing liquid quantities of 1 to 200 times, preferably of 10 to 100 times the own weight of the composition.

Another method for preparing the compositions according to the invention consists of applying the cholesteryl sulfate onto the carrier material, or of coating or impregnating the carrier material with the cholesteryl sulfate. Coating can be carried out by, for example, spraying or printing a solution of cholesteryl sulfate onto the carrier material, or by squeegeeing a solution of cholesteryl sulfate on the carrier material, followed by a drying step for removing the solvent. Preferably, coating is carried out by spray coating, for example by means of air brush technique with a solution, preferably a methanolic solution of cholesteryl sulfate.

Impregnating can be carried out by, for example, immersing or steeping the carrier material in a solution of cholesteryl sulfate or by dripping a solution of cholesteryl sulfate onto it, optionally followed by a drying step for removing the solvent. Preferably, carrier materials based on collagen sheets preferably present in a freeze-dried form, and conventional wound dressings are coated or impregnated with cholesteryl sulfate by means of spray coating. In the process, methanolic solutions having for example concentrations of 0.1 % by wt to 1.2% by wt of cholesteryl sulfate are sprayed onto the carrier materials, so that compositions containing cholesteryl sulfate in quantities of for example from 0.01 % by wt to 10% by wt relative to the total weight of the composition are obtained.

In another preferred embodiment, carrier materials, such as, e.g., preferably freeze-dried collagen sheets are steeped in an aqueous vesicle solution of cholesteryl sulfate. In the process, preferably 0.1 to 20 ml, more preferably 0.5 to 5 ml of a 0.01 to 5% by wt, more preferably of a 0.1 to 2% by wt aqueous solution of cholesteryl sulfate, which, for example, can be prepared according to the above-described method, is employed per 1 cm³ carrier material. After steeping and the complete liquid absorption by the carrier material, the latter is subjected to drying, particularly preferably freeze-drying.

The weight of the compositions according to the invention per dosage unit generally is from 10 mg to 6 g, preferably from 20 mg to 600 mg, still more preferably from 50 mg to 300 mg. In designs in the form of, for example, sheets, layers or fleeces, the length and width of the composition are at least ten times, preferably at least 20 times the size of the thickness, they can be cut or punched into or provided with a stamp, and have surfaces of preferably at least about 25 cm², more preferably of at least about 50 cm², still more preferably of at least 100 cm².

It was found that cholesteryl sulfate is exceptionally well suited of being applied onto carrier materials, especially onto carrier material in the form of layers, sheets, matrices, pads or foams, such as, for example, collagen, alginate or chitosan sheets or other conventional wound dressing materials or of being used as a haemostatic device having an optimal application facility when worked into them.

Application of the cholesteryl sulfate onto the carrier materials used according to the invention furthermore can lead to an enhancement of the haemostatic effect of the cholesteryl sulfate. Especially the combination of a carrier material which itself acts as a haemostatic with cholesteryl sulfate is suitable to provide haemostatic devices with improved blood clotting activity. In this context it has to be pointed out, that in order to start the coagulation cascade, the strongest possible activation of the primary triggering processes is therefore generally attempted. This means, with regard to the combination of cholesteryl sulfate with haemostatic carrier materials, however, a synergistic effect cannot obviously be assumed. Much more, it is to be expected that a competitive situation rather occurs and that the more effective stimulant makes a contribution in triggering the coagulation cascade, whereas the less active substance does not become synergistically active but rather exhibits no involvement.

Moreover, it may occur that a synergistic effect of substances in isolated partial systems may be observed but that it does not play any role with regard to efficiency in blood coagulation. In this respect, the work by Cvern et al. (2007) should be mentioned, for example. In this case, it was possible to show that a combination of thrombin or tissue factor and collagen in fact has a synergistic effect on thrombocyte aggregation, but that it does not entail any measurable difference between the individual or combined effect of the two reagents in the assessment of the total effect on the coagulation of blood in the thromboelastogramm.

Under these aspects, it becomes clear that, in the case of two stimulants which are effective individually, no direct conclusion can be drawn that they exhibit an improved effect when combined.

It is common knowledge, that the speed of blood coagulation is increased due to the presence of negative surface charges. Cholesteryl sulphate at ambient pH also exhibits a negative charge, which contributes to blood coagulation accelerating properties of this material. Carrier materials according to our findings can also be charged positively, in particular is this case for acidic collagen and chitosan. Surprisingly it was found, that although the surface charge of the carrier material and the cholesteryl sulphate has opposite direction, the ability to accelerate blood coagulation is even increased by the combination of carrier material and the cholesteryl sulphate.

In the case of the compositions of the present invention comprising cholesterol sulfate and a carrier material with haemostatic activity, however, such a synergy or enhanced coagulation effect can be observed surprisingly.

In addition, such cholesteryl sulfate-containing carrier compositions are characterized, in particular, by the great variability and configuration possibilities of the carrier material, so that these can be optimally adapted to the location of application or the type of application required. For example, the use of the composition according to the invention of cholesteryl sulfate and at least one carrier material for a slight to medium exterior or superficial haemostasis requires a different carrier material from, for example, heavily bleeding wounds or internal haemorrhage. Owing to the great variability of the carrier materials, topical application systems can be provided both for external as well as internal use for haemostasis. For example, the use of simpler or thinner and lighter dressing material is conceivable, such as, for example, simple stable and elastic bandages, for example from cotton or mull gauze, absorbent compresses, or even merely such material in the form of a plaster, which, owing to the external and superficial application, can be easily removed once the haemostasis is completed. Heavy bleeding, however, potentially requires treatment with thick and highly absorbent dressing materials. Moreover, haemorrhage can also occur internally; in the case of operation, especially, it is often necessary to stop heavy internal haemorrhage quickly and efficiently. Here, special requirements must be made of haemostatics for internal application. For example, such internal haemorrhages, for example operation wounds, require treatment with a biocompatible material which can preferably remain at the site of the wound and is metabolised there, after coagulation and clot formation has set in. Such special biocompatible and degradable wound dressing materials which remain in a wound and are not removed again, fall into the group of implants: In particular, carrier materials based on collagen are particularly suitable as implants. Collagen, as a natural constituent of human or animal tissue, has a great compatibility and biocompatibility with the environmental parameters prevailing in a wound. In the course of natural wound healing and endogenic metabolic activities, the carrier material is slowly and gently degraded during the further healing process. The blood clot formed in or on the wound, respectively, or the material clotted and solidified in the carrier material on the wound, respectively, which leads to wound closure and, thus, to a stop of the haemorrhage owing to this coagulation and solidification, can remain on the wound as a natural closure of the wound. Materials that are not biocompatible or degradable in the body, however, cannot be used as an implant. After the haemostasis has set in, they must be removed from the wound or the site of the operation. This entails the danger of the blood clot sticking to the wound being torn off, thus ripping the wound open again. This clearly shows the advantage of a biocompatible, degradable carrier substance, in particular in this special form of application of the implants.

Moreover, cholesteryl sulfate, in particular in compositions that additionally contain at least one carrier material as well as, optionally, further active agents and auxiliary substances, is well-suited for pharmaceutical application in so far as the material or compositions do not exhibit any loss of activity worth mentioning, even after sterilisation. A gamma sterilisation, in particular, may be used for the compositions according to the invention.

Storage of the compositions also does not negatively affect the stability.

The special haemostatic effect of cholesteryl sulfate as well as of compositions of cholesteryl sulfate with at least one carrier material will be illustrated by the following examples. These examples prove the extraordinary suitability of cholesteryl sulfate as a haemostatic. The combination with various carrier materials also exhibits good properties as regards haemostasis. Thus, cholesteryl sulfate as such, as well as in compositions containing at least one carrier material, is particularly suitable as a haemostatic.

### EXAMPLES

### 1. Effect of Sodium Cholesteryl Sulfate (SCS) in Porcine Whole Blood

The haemostatic effect of cholesteryl sulfate as such was investigated using a Sonoclot® Coagulation & Platelet Function Analyzer by Scienco® Inc with regard to the clotting time (ACT) and clot rate. In this case, the ACT value indicates the time until the onset of the first thickening of the blood, which matches the time required by the system for generating a first blood clot. The clot rate indicates the speed with which the clot solidifies, and is there fore a measure for fibrin formation.

For the investigations presently illustrated, different quantities of cholesteryl sulfate in the form of its sodium salt was dissolved in methanol, and aliquots were transferred into the reaction vessels. By evaporating the methanol, the cholesteryl sulfate was precipitated as a layer on the bottom of the reaction vessel. Then 360 µl of porcine citrate blood were put into the reaction vessel, and 20 µl 0.2 M calcium chloride solution were subsequently added for recalcification. Both the time until coagulation (ACT) as well as the clot formation rate were measured with the Sonoclot Analyzer.

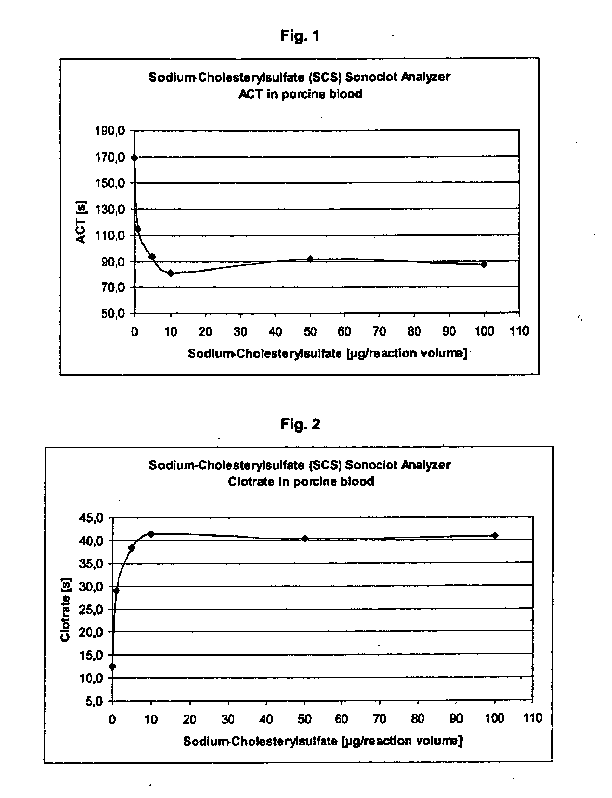

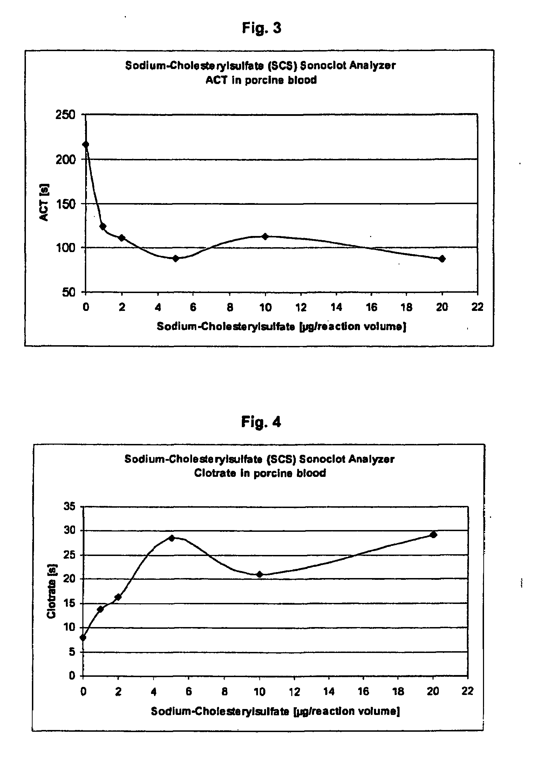

The experiment was carried out with two different samples of porcine blood. The results of the above tests are shown in Figures 1 to 4.

### 2. Effect of Sodium Cholesteryl Sulfate (SCS) in Collagen Carrier Material in Porcine Whole Blood

In addition, the haemostatic effect of a composition containing cholesteryl sulfate in a carrier material of collagen was investigated, which is obtainable in accordance with the method preferred according to the invention, comprising the steps of:
1. preparing an aqueous collagen suspension
2. admixing
   a) an aqueous vesicle solution of cholesteryl sulfate
   b) with gelatine particles coated with cholesteryl sulfate
3. pouring the mixture into a mould
4. freezing the mixture in the mould and freeze-drying the mixture while the composition according to the invention is formed.

The compositions thus prepared where investigated with regard to the clot formation time of recalcified porcine blood using a so-called "HOWELL clotting test", a modification of the "determination of the recalcification time in whole blood according to HOWELL" (W. Rick, Klinische Chemie und Mikroskopie, 5. ed, Springer Verlag, Berlin, p 110). Here, the HOWELL test was modified by using plastic vessels instead of glass vessels. The indicated cholesteryl sulfate contents represent % by wt relative to the finished freeze-dried total composition.

### a) admixing an aqueous vesicle solution

In order to prepare an aqueous vesicle solution, sodium cholesteryl sulfate was suspended in water and exposed to ultrasound (device Sonoplus by Bandelin) by means of a sonotrode at a constant temperature of 85°C for 4 x 1 minute at 50 watts, so that a stable vesicle solution was formed.

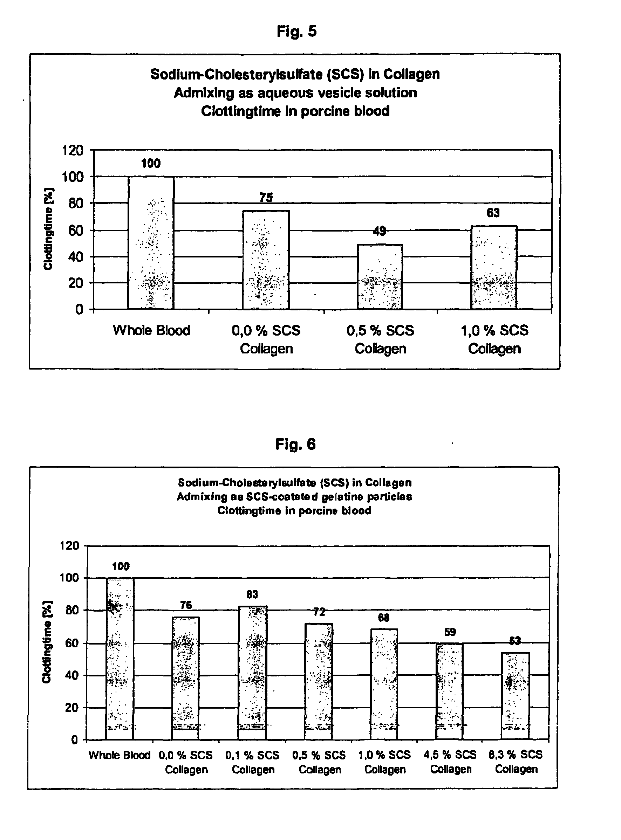

Aqueous vesicle solutions of varying concentrations were admixed into collagen suspension as known from the prior art, such as, for example, DE 4028622, and homogenised, so that compositions having Na-cholesteryl sulfate contents of 0.5% and 1.0%, relative to the finished composition, were obtained. The clotting rates in the "HOWELL clotting test" of these materials are shown in Fig. 5.

### b) Admixing of gelatine particles coated with cholesteryl sulfate

Another method of introducing cholesteryl sulfate consists of applying the cholesteryl sulfate onto an inert carrier substance and subsequently working the latter into the solution or suspension of the carrier material. Particularly when using collagen as a carrier mass, gelatine particles onto which cholesteryl sulfate can be brought in a simple manner are particularly suitable. For this purpose, a methanolic solution of sodium cholesteryl sulfate (SCS) is added to gelatine particles or a gelatine powder. Then, the methanol is evaporated, and SCS is thus brought onto the particles. Here, the maximum loading density is 50% by wt relative to the coated particles, so that the coated gelatine is present in a weight ratio gelatine:SCS of 2:1. The particles coated thus can be easily and homogeneously worked into the collagen mass. The results of the "HOWELL clotting test" of compositions thus prepared having Na cholesteryl sulfate contents of 0.1 to 8.3 %, relative to the finished composition, is shown in Fig. 6.

### HOWELL Clotting Test:

### Chemicals:

### Citrate Buffer (for blood preservation, inhibits early clotting of the blood):

Trisodium Citrate Dihydrate: 1,6 g / 50 ml deionized water

In a PE-vial with screw cap suitable for storing 500 ml of whole blood

### Calcium Chloride Hexahydrate:

4,38 g /100 ml deionized water

### Equipment:

Water bath (37 °C)
Sterile Falcon reaction vessels (15 ml)
Sterile diluting loop
Tweezers
Punch (1 cm diameter)
Hammer
Teflon-coated board
Pipette stepper
Pipette (Eppendorf) 100-1000 µl
PE-vial with screw cap (500 ml)

### Test Execution:

Fresh porcine blood is collected in PE-vial with screw cap containing 50 ml citrate buffer. Citrate acts as a calcium-catcher and prohibits early clotting of the blood.

3,5 ml whole blood are filled into the reaction vial with the pipette stepper. The blood sample is incubated in the water bath at 37 °C for 5 minutes.

With the punch pieces of 1 cm diameter are punched from the compositions and carrier materials for the tests.

### Recalcification:

For recalcification the tempered whole blood is mixed with 0.2 ml calcium chloride solution.

With the diluting loop clotting is initiated by moving the loop up and down in the blood containing test vial and blood is kept flowing. The time from addition of calcium chloride solution until clotting of the blood is measured.

The same procedure is executed with the samples wherein 2 pieces (1cm diameter) of the compositions or carrier materials are added to the whole blood directly before the addition of calcium chloride solution.

The time from addition of calcium chloride solution until clotting is compared.

### 3. Effect of Carrier Materials coated with Sodium Cholesteryl Sulfate (SCS) in Porcine Whole Blood and in Human Blood

Another method for preparing the compositions according to the invention consists of applying the cholesteryl sulfate onto the desired carrier material.

Apart from impregnating methods such as, for example, dipping, steeping or dripping, coating methods such as spray coating by means of an airbrush technique or other printing methods are also suitable.

In a preferred embodiment, freeze-dried collagen sheets were impregnated with an aqueous cholesteryl sulfate vesicle solution.

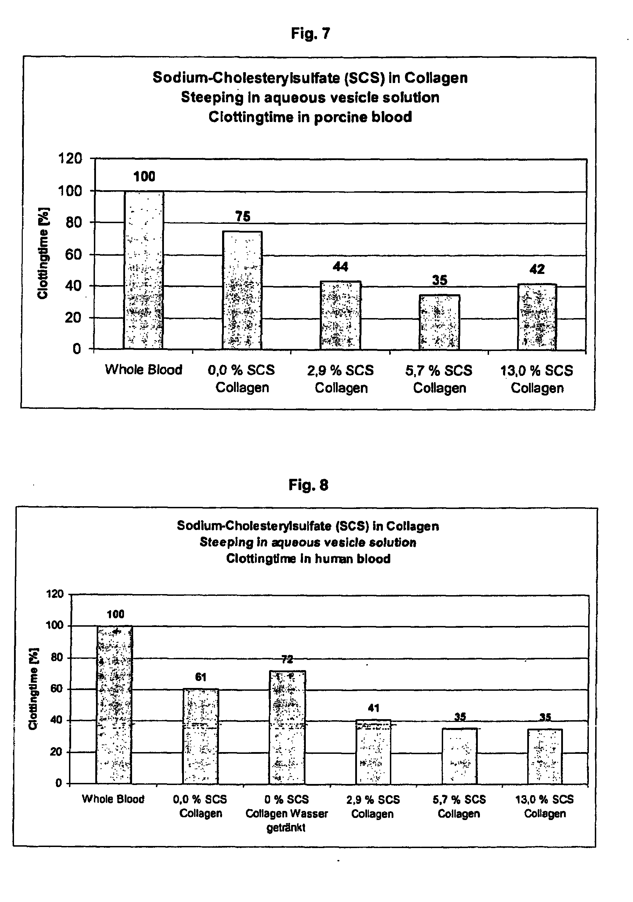

To this end, an aqueous vesicle solution is prepared as described above, and the collagen sheet is steeped in it, with 1 ml vesicle solution of varying concentrations per 1 cm³ carrier matrix being used. The steeped sheet is then subjected to another freeze-drying process. The results in the "HOWELL clotting test" of collagen sheets thus prepared, with Na-cholesteryl sulfate contents of 2.9 to 13%, relative to the finished composition in porcine blood, is shown in Figure 7. The results of the clotting times in human whole blood is shown in Figure 8.

In another preferred embodiment, the cholesteryl sulfate was applied onto the desired carrier material by spray coating. Since sodium cholesteryl sulfate (SCS) is soluble in highly volatile methanol, this offers a good option of spraying carrier materials, such as, for example, conventional wound dressings or freeze-dried collagen, alginate or chitosan sheets with this solvent-active-agent-mixture. Particularly in the embodiment preferred according to the invention of the spray coated collagen sheets, this method could be applied extraordinarily well, without any occurrence of a strong change of the surface or a collapse of the structure of the sheet material.

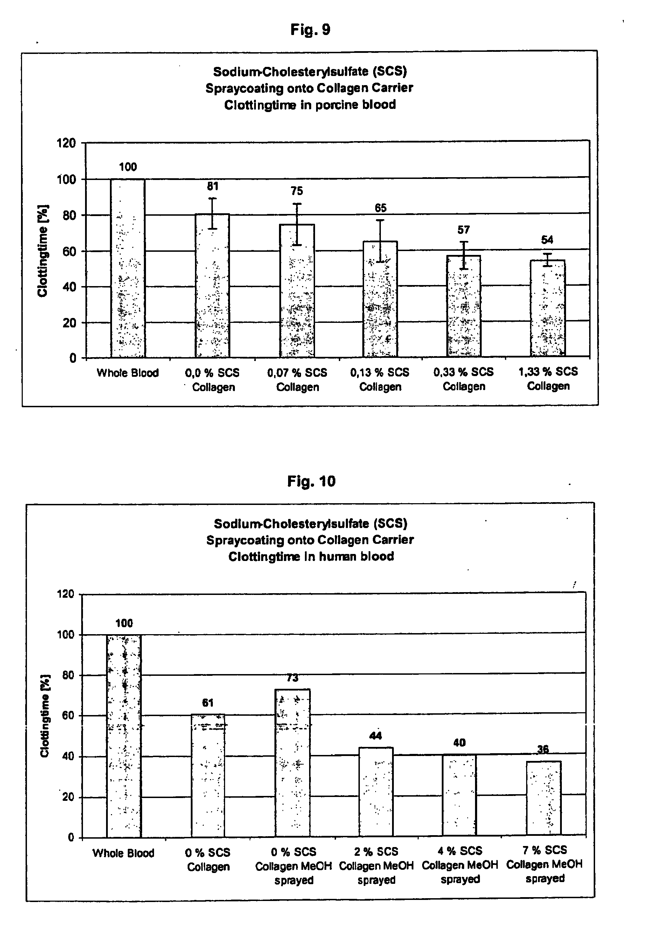

For such a preferred embodiment of a spray-coated collagen sheet, collagen sheets obtainable in accordance, for example, with the prior art, for example, DE 4028622, having a thickness of 2 mm, are fixed on a carrier and sprayed with 4 to 6 ml methanolic SCS solution and dried completely at room temperature. In this manner, quantities of 0.5 to 110 mg SCS were applied to sheets of a size of 210 x 148 mm, a thickness of 2 mm and an average weight of 1.5 g per sheet. Thus, there was an SCS load of 0.03 to 7% by wt, relative to the total composition. The coated collagen materials thus obtained were investigated with respect to their clotting performance by means of the "HOWELL clotting test". Some results regarding the clotting time are shown in Fig. 9. Fig. 10 shows results of the clotting times in human whole blood.

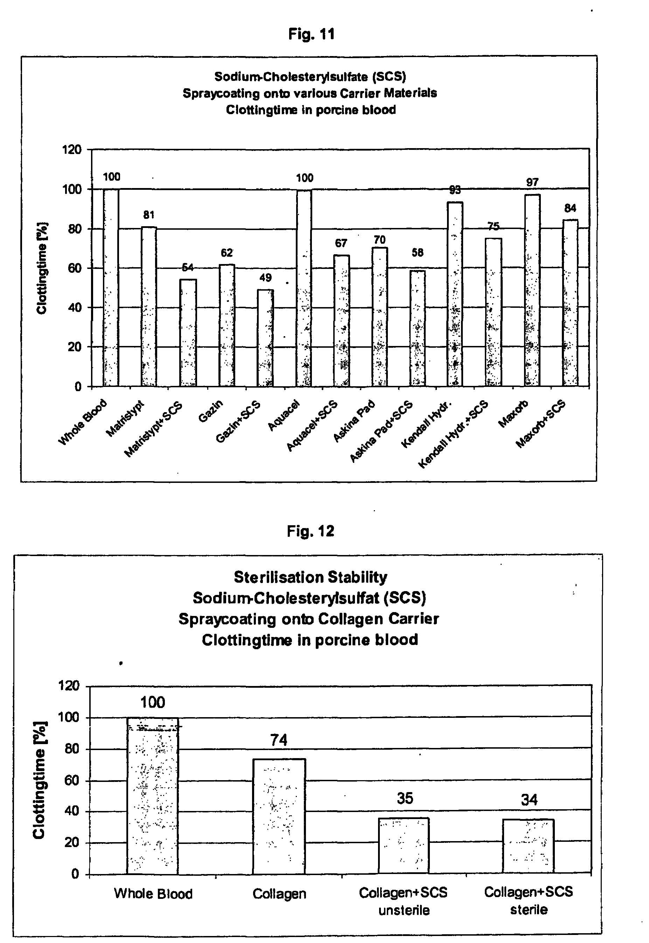

Other carrier materials, in particular conventional wound dressings, can also be coated with SCS using this method. Here, impregnation can be carried out not only by means of spray coating, but also, for example, by dripping an SCS-containing solution onto the carrier materials and subsequent drying at room temperature of the carrier materials thus coated or impregnated The coagulation effect of various coated or impregnated carrier materials is compared in Figure 11. Here, the following materials were equipped with a concentration of 6.7 mg SCS per 100 cm² and investigated as regards their clotting performance in porcine blood as compared to whole blood, as well as to the respective uncoated material.

| **Brand name** | **Manufacturer** | **Composition** | **Impregnation** |
|---|---|---|---|
| Matristypt^{®} | Dr. Suwelack Skin & Health Care AG | Collagen (Sheet) | Spray coating |
| Gazin^{®} | Lohmann & Rauscher | Mull, gauze (Compress) | Dripping / Spray coating |
| Aquacel^{®} | Convatec | Sodium carboxymethylcellulose (sheet) | Spray coating |
| Askina Pad^{®} wound dressing | B. Braun | Cotton/acrylic fibres (compress) | Dripping |
| Kendall Hydrafoam^{®} | Tyco Healthcare | Polyurethane (sponge) | Dripping |
| Maxorb^{®} extra | Medline | Sodium carboxymethylcellulose / calcium alginate (sheet) | Spray coating |
| Teflon | Olifan | PTFE-band (poly-tetra-fluorethylene) | Spray coating |

### 4. Sterilisation Stability

Furthermore, the materials according to the invention are stable when subjected to sterlisation treatments. In this regard, Figure 12 shows results from the "HOWELL clotting test" on the coagulation effect of collagen carrier materials that were coated by means of spray coating with 1 % by wt (relative to the total composition) or 6.4 mg/100 cm² Na-cholesteryl sulfate and γ-sterilised at 20 kGy.

### 5. Enhanced Effects / Synergism

### 5.1 Sonoclot-Analysis of Collagen with SCS in Porcine Whole Blood

In order to show the enhanced effects in blood clotting of the combination of cholesteryl sulphate as a haemostatic agent and a haemostatic carrier material such as collagen the Sonoclot-Test has been carried out according to example 1.

The test condition and materials were in accordance with example 1.

As a carrier material freeze-dried, milled collagen from the company Dr. Suwelack Skin & Health Care AG (SHC) as well as collagen from bovine tendon from the company Sigma Aldrich have been examined in the Sonoclot-Analyzer and compared to cholesteryl sulphate as well as to a combination of the collagen and cholesteryl sulphate (SCS).

For the examination of the collagen carrier material 1,1 mg carrier material were transferred into the reaction vessels containing 360 µl porcine whole blood. 12,5 µl isosmotic saline solution (NaCl) were subsequently admixed and 20 µl 0.2 M calcium chloride solution were subsequently added for recalcification. Both the time until coagulation (ACT) as well as the clot formation rate were measured with the Sonoclot Analyzer. High clot rate indicates fast fibrin formation and thus fast clot formation.

For the examination of the collagen carrier material in combination with cholesteryl sulphate instead of isosmotic saline solution 12,5 µl of a choesteryl sulphate vesicle solution containing 4 % by wt cholesteryl sulphate is admixed with the carrier material.

For the examination of the cholesteryl sulphate a 4 % by wt vesicle solution alone was tested accordingly.

The vesicle solution was prepared as described in example 2, Nr. 2 a).

Results are as follows:

| | **Experiment 1** | | **Experiment 2** | |
|---|---|---|---|---|
| | **ACT [s]** | **clot rate** | **ACT [s]** | **clot rate** |
| **Whole blood** | **336** | **6** | **276** | **9** |
| **12,5 µl SCS** | **90** | **17** | **81** | **29** |
| **Collagen (SHC) + 12,5µl NaCl** | **208** | **10** | **178** | - |
| **Collagen (SHC) + 12,5µl SCS** | **63** | **39** | **63** | - |
| **bovine tendon + 12,5µl NaCl** | - | - | **163** | **11** |
| **bovine tendon + 12,5µl SCS** | - | - | **61** | **47** |

### 5.2 Howell Clotting Analysis of Collagen Spray Coated with SCS in Porcine Whole Blood

Investigations showing enhanced clotting activity of carrier materials which were spray coated with SCS have been carried out in the Howell-Test as described in Example 2.

The preparation of the spray coated carrier materials was carried out as described in Example 3.

SCS was spray coated onto the carrier materials in a) single and b) threefold amount. Furthermore SCS alone was tested in an amount according to a) and b) per test vessel. For each test vial 2 pieces of 1 cm diameter each were cut from the coated material. Therewith each test vial contained either a carrier material with a) the single or b) the threefold amount of SCS coated on the carrier materials respectively.

The preparation of the SCS coated test vial was carried out by preparing a solution of SCS in methanol and evaporating the methanol with gaseous nitrogen from the rotating reaction vessel. Therewith SCS coated test vials with similar amounts a) and b) per test vial were achieved.

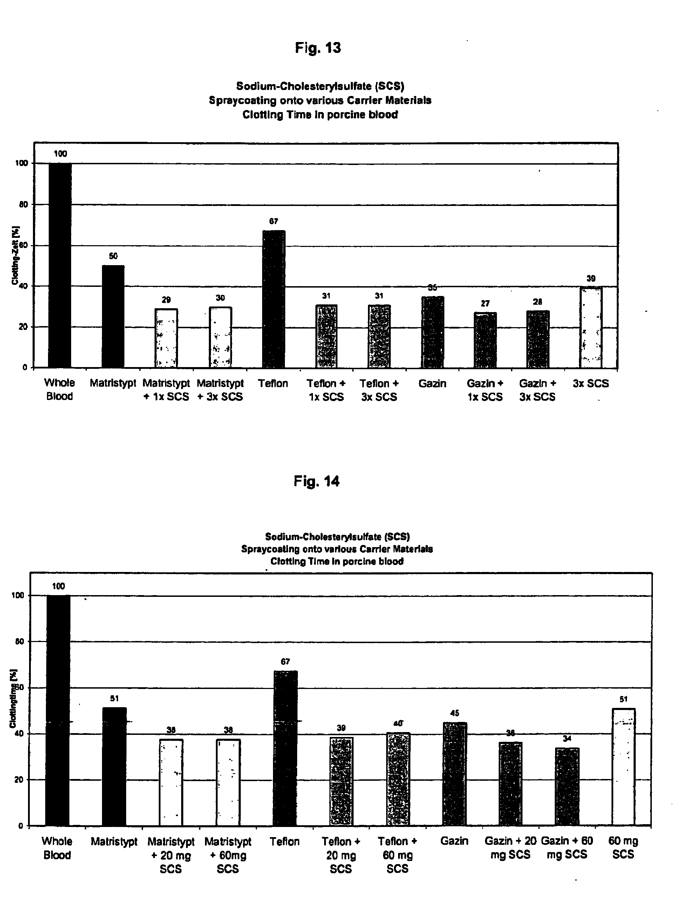

Results are shown in Figure 13.

### 5.3 Howell Clotting Analysis of Collagen Spray Coated with SCS in Porcine Whole Blood

Investigations showing enhanced clotting activity of carrier materials which were spray coated with SCS have been carried out in the Howell-Test as described in Example 2.

The preparation of the spray coated carrier materials was carried out as described in Example 3.

The amount of SCS spray coated onto the carrier materials is 20 mg or 60 mg SCS per DIN A5 sheet respectively, which is in accordance with 0,104 mg or 0,314 mg SCS per test vessel. For each test vial 2 pieces of 10 mm diameter and 1 mm thickness each were cut from the coated material. Therewith each test vial contained an amount of 0,104 mg or 0,314 mg SCS respectively coated on the carrier material.

The preparation of the SCS coated test vial comprising 0,314 mg SCS was carried out as described in Example 5.2

Results are shown in Figure 14.

In all experiments shown here, attempts were made to avoid a pre-activation of the samples used so that an activation of the coagulation or its acceleration can be measured only starting with the contact of the blood samples. In this regard, it must be remarked that, surprisingly, it was found that
1.) cholesterol sulfate has a coagulation-promoting effect also in a non-activated system (which is in contrast to the literature cited above), and
2.) that no pre-incubation with cholesterol sulfate is required in order to initiate fast coagulation.

The results of the experiments especially those carried out under Example 5 show, that SCS or the carrier materials alone exhibit weaker coagulation activity than the combination of SCS with the carrier.

In this context it has to be referred to the above made explanations regarding the biochemical blood clotting cascade and it has therefore to be pointed out that a linear synergistic effect can not be expected. Rather in the case of two stimulants (cholesteryl sulfate and carrier) which are effective individually, no direct conclusion can be drawn that they exhibit an improved effect when combined. Nevertheless the results of the Example 5 clearly show such unexpected enhanced effects for compositions containing cholesteryl sulfate and a carrier material compared to the carrier material or the cholesteryl sulfate alone.

### Literature

Blache D., Becchi M., Davignon J.: Occurence and biological effects of cholesteryl sulfate on blood platelets; Biochemica et Biophysica Acta, 1995 (1259), 291-296.
Iwamori M., Iwamori Y., Ito N.: Regulation of the Activities of Thrombin and Plasmin by Cholesterolsulfate as a Physiological Inhibitor in Human Plasma; J. Biochem, 1999 (125), 594-601
Merten M., Dong J. F., Lopez J. A.: Cholesterol Sulfate - A New Adhesive Molecule for Platelets; Circulation, 2001 (103), 2032-2034
Shimada T., Kato H., lwanaga S., lwamori M., Nagai Y.: Activation of factor XII and prekallikrein with cholesterol sulfate; Thrombosis Research, 1985 (38), 21-31
Strott C. A., Higashi Y.: Cholesterol sulfate in human physiology - what's it all about; Journal of lipid research, 2003 (44), 1268 - 1278
Cvern G. et. al. : Collagen / endogenous thrombin-induced platelet aggregation in whole blood samples; Blood Coagulation and Fibinolysis; 2007 (18), 585-588

### Explanations of the Figures:

Figure 1:
   Test 1: Clotting time of porcine blood with Na-cholesteryl sulfate (ACT in Sonoclot)
Figure 2:
   Test 1: Clot formation rate of porcine blood with Na-cholesteryl sulfate (clot rate in Sonoclot)
Figure 3:
   Test 2: Clotting time of porcine blood with Na-cholesteryl sulfate (ACT in Sonoclot)
   Figure 4:
Test 2: Clot formation rate of porcine blood with Na-cholesteryl sulfate (clot rate in Sonoclot)
Figure 5:
   Clotting time of porcine blood with compositions of Na-cholesteryl sulfate in collagen carrier material prepared by admixing an aqueous vesicle solution of cholesteryl sulfate into the carrier material (HOWELL clotting test)
Figure 6:
   Clotting time of porcine blood with compositions of Na-cholesteryl sulfate in collagen carrier material prepared by admixing gelatine particles coated with cholesteryl sulfate into the carrier material (HOWELL clotting test)
Figure 7:
   Clotting time of porcine blood with compositions of collagen carrier materials steeped in. Na-cholesteryl sulfate vesicle solutions and freeze-dried again (HOWELL clotting test)
Figure 8:
   Clotting time of human whole with compositions of collagen carrier materials steeped in Na-cholesteryl sulfate vesicle solutions and freeze-dried again (HOWELL clotting test)
Figure 9:
   Clotting time of porcine blood with compositions of collagen carrier materials spray coated with Na-cholesteryl sulfate (HOWELL clotting test)
Figure 10:
   Clotting time of human whole blood with compositions of collagen carrier materials spray coated with Na-cholesteryl sulfate (HOWELL clotting test)
Figure 11:
   Clotting time of porcine blood with compositions of wound dressing materials coated or impregnated with Na-cholesteryl sulfate (HOWELL clotting test)
Figure 12:
   Clotting time of porcine blood with compositions of collagen carrier materials spray coated with Na-cholesteryl sulfate with and without γ-sterilisation (HOWELL clotting test)
Figure 13:
   Clotting time of porcine blood with selected compositions of carrier materials coated with Na-cholesteryl sulfate in comparison to the carrier materials or Na-cholesteryl sulphate respectively (HOWELL clotting test)
Figure 14:
   Clotting time of porcine blood with selected compositions of carrier materials coated with Na-cholesteryl sulfate in comparison to the carrier materials or Na-cholesteryl sulphate respectively (HOWELL clotting test)

## Claims

1. Composition containing cholesteryl sulphate and at least one carrier material in the form of a sheet, layer, pad, film, foam, fleece, compress or plaster for use as a haemostatic agent.

2. Composition for use according to claim 1, wherein the haemostatic agent is for topical and/or external haemostasis or for use as an implant.

3. Composition for use according to any one of the claims 1 to 2. wherein the carrier material is selected from natural and/or synthetic polymers or mixtures thereof, especially from the group of polysaccharides, glucosaminoglycanes, proteins and/or synthetic polymers or mixtures thereof.

4. Composition for use according to any one of the claims 1 to 3, wherein the carrier material is collagen or a conventional wound dressing.

5. Composition for use according to any one of the claims 1 to 4, wherein the cholesteryl sulphate is sodium cholesteryl sulphate.

6. Composition for use according to any one of the claims 1 to 5, which additionally contains one or more further active agents and/or one or more auxiliary substances.

7. Composition for use according to any one of the claims 1 to 6, having a homogeneous distribution of the ingredients in the carrier material.

8. Composition for use according to any one of the claims 1 to 6, wherein the cholesteryl sulphate is applied onto the carrier material, especially by spray coating or by steeping the carrier material in a cholesteryl sulphate-containing solution or by steeping the carrier material in an aqueous vesicle solution of cholesteryl sulphate.

9. Composition for use according to any one of the claims 1 to 8, which is present in the form of a freeze-dried matrix composition.

10. Composition for use according to any one of the claims 1 to 9, containing
- at least 50% by wt of carrier material(s),
- at least 0.03 % by wt of cholesteryl sulphate,
- 0 to 40% by wt of one or more further active agents
- 0 to 40% by wt of one or more auxiliary substances, and
- less than 25% by wt of water,
in each case relative to the total quantity of the composition.

11. Method for preparing a composition for use according to any one of the claims 1 to 7 and 9 to 10, which method comprises the steps:
1. preparing an aqueous solution or suspension of at least one carrier material,
2. admixing of cholesteryl sulphate and, optionally, one or more active agents and/or auxiliary substances,
3. pouring the mixture from step 2, and
4. drying the mixture.

12. Method according to claim 11, wherein, in step 2, the cholesteryl sulphate is admixed in the form of an aqueous vesicle solution or in the form of carrier particles coated with cholesteryl sulphate, especially gelatine particles coated with cholesteryl sulphate.

13. Method according to any one of the claims 11 to 12, wherein step 4 comprises pouring into a mould, and step 5 comprises freezing the mixture in the mould and freeze-drying the mixture.

14. Method for preparing a composition for use according to any one of the claims 1 to 6 and 8 to 10, which method comprises applying cholesteryl sulphate onto a layer-like carrier material, especially by spray coating a layer-like carrier material or by steeping the carrier material in a cholesteryl sulphate-containing solution and subsequent drying.

## Patentansprüche

1. Zusammensetzung enthaltend Cholesterylsulfat und mindestens ein Trägermaterial in Form von einem Blatt, einer Schicht, eines Kissens, eines Films, eines Schaums, eines Vlieses, einer Kompresse, oder eines Pflasters zur Verwendung als ein hämostatisches Mittel.

2. Zusammensetzung zur Verwendung nach Anspruch 1, wobei das hämostatische Mittel zur topischen Hämostase und/oder äußeren Hämostase oder zur Verwendung als ein Implantat gedacht ist.

3. Zusammensetzung zur Verwendung nach einem der Ansprüche 1 bis 2, wobei das Trägermaterial aus natürlichen und/oder synthetischen Polymeren oder Gemischen davon, insbesondere aus der Gruppe von Polysacchariden, Glucosaminoglycanen, Proteinen und/oder synthetischen Polymeren oder Gemischen davon ausgewählt ist.

4. Zusammensetzung zur Verwendung nach einem der Ansprüche 1 bis 3, wobei das Trägermaterial Collagen oder ein herkömmlicher Wundverband ist.

5. Zusammensetzung zur Verwendung nach einem der Ansprüche 1 bis 4, wobei das Cholesterylsulfat Natriumcholesterylsulfat ist.

6. Zusammensetzung zur Verwendung nach einem der Ansprüche 1 bis 5, die zusätzlich einen oder mehrere Wirkstoff und/oder eine oder mehrere Hilfssubstanzen enthält.

7. Zusammensetzung zur Verwendung nach einem der Ansprüche 1 bis 6, mit einer homogenen Verteilung der Inhaltsstoffe in dem Trägermaterial.

8. Zusammensetzung zur Verwendung nach einem der Ansprüche 1 bis 6, wobei das Cholesterylsulfat auf das Trägermaterial aufgebracht wird, insbesondere durch Sprühbeschichten oder durch Quellenlassen des Trägermaterials in einer Lösung, die Cholesterylsulfat enthält, oder durch Quellenlassen des Trägermaterials in einer wässrigen Vesikel-Lösung von Cholesterylsulfat.

9. Zusammensetzung zur Verwendung nach einem der Ansprüche 1 bis 8, die in Form einer gefriergetrockneten Matrix-Zusammensetzung vorhanden ist.

10. Zusammensetzung zur Verwendung nach einem der Ansprüche 1 bis 9, enthaltend
- mindestens 50 Gew.-% Trägermaterial(ien),
- mindestens 0,03 Gew.-% Cholesterylsulfat
- 0 bis 40 Gew.-% von einem oder mehreren zusätzlichen Wirkstoffen,
- 0 bis 40 Gew.-% von einem oder mehreren zusätzlichen Hilfssubstanzen,
und
- weniger als 25 Gew.-% Wasser,
in jeden Fall in Bezug auf die Gesamtmenge der Zusammensetzung.

11. Verfahren zur Herstellung einer Zusammensetzung zur Verwendung nach einem der Ansprüche 1 bis 7 et 9 bis 10, wobei das Verfahren die folgenden Schritte umfasst:
1. Herstellen einer wässrigen Lösung oder einer Suspension von mindestens einem Trägermaterial,
2. Mischen mit Cholesterylsulfat und, gegebenenfalls, einem oder mehreren Wirkstoffen und/oder Hilfssubstanzen,
3. Gießen des Gemisches von Schritt 2, und
4. Trocknen des Gemisches.

12. Verfahren nach Anspruch 11, wobei, in Schritt 2, das Cholesterylsulfat in Form einer wässrigen Vesikel-Lösung oder in Form von mit Cholesterylsulfat überzogenen Trägerteilchen, insbesondere mit Cholesterylsulfat überzogenen Gelatineteilchen gemischt wird.

13. Verfahren nach einem der Ansprüche 11 bis 12, wobei der Schritt 4 das Gießen in eine Form und der Schritt 5 das Tiefgefrieren des Gemisches in der Form und das Gefriertrocknen des Gemisches umfasst.

14. Verfahren zur Herstellung einer Zusammensetzung zur Verwendung nach einem der Ansprüche 1 bis 6 et 8 bis 10, wobei das Verfahren das Aufbringen von Cholesterylsulfat auf ein schichtartiges Trägermaterial, insbesondere durch Sprühbeschichten eines schichtartigen Trägermaterials oder durch Quellenlassen des Trägermaterials in einer Cholesterylsulfat-enthaltenden Lösung, und anschließend das Trocken umfasst.

## Revendications

1. Composition contenant du sulfate de cholestéryle et au moins un matériau porteur en forme d'une feuille, une couche, un coussin, un film, une mousse, un molleton, une compresse ou un plâtre pour l'utilisation à titre d'un agent hémostatique.

2. Composition pour l'utilisation selon la revendication 1 dans laquelle l'agent hémostatique est pour l'hémostase topique et/ou externe ou pour l'utilisation à titre d'un implant.

3. Composition pour l'utilisation selon l'une des revendication 1 à 2, dans laquelle le matériau porteur est sélectionné parmi les polymères naturels et/ou synthétiques ou les mélanges de ceux-ci, en particulier parmi le groupe des polysaccharides, glucosaminoglycanes, protéines et/ou des polymères synthétiques ou des mélanges de ceux-ci.

4. Composition pour l'utilisation selon l'une des revendications 1 à 3, dans laquelle le matériau porteur est le collagène ou un pansement de blessure conventionnel.

5. Composition pour l'utilisation selon l'une des revendications 1 à 4, dans laquelle le sulfate de cholestéryle est le cholestéryle sulfate de sodium.

6. Composition pour l'utilisation selon l'une des revendications 1 à 5, qui contient additionnellement un ou plusieurs agents actifs et/ou un ou plusieurs substances auxiliaires.

7. Composition pour l'utilisation selon l'une des revendications 1 à 6, ayant une distribution homogène des ingrédients dans le matériau porteur.

8. Composition pour l'utilisation selon l'une des revendication 1 à 6, dans laquelle le sulfate de cholestéryle est appliqué sur le matériau porteur, en particulier par revêtement par pulvérisation ou par laisser tremper le matériau porteur dans une solution contenant du sulfate de cholestéryle ou par laisser tremper le matériau porteur dans une solution vésicule aqueuse de sulfate de cholestéryle.

9. Composition pour l'utilisation selon l'une des revendications 1 à 8, qui est présente en forme d'une composition matrice lyophilisée.

10. Composition pour l'utilisation selon l'une des revendications 1 à 9, contenant
- au moins 50 % en poids de matériau(x) porteur(s),
- au moins 0,03 % en poids de sulfate de cholestéryle,
- 0 à 40 % en poids de l'un ou plusieurs d'agents actifs additionnels,
- 0 à 40 % en poids de l'un ou plusieurs de substances auxiliaires, et
- moins de 25 % en poids d'eau,
en chaque cas en relation à la quantité totale de la composition.

11. Méthode de préparation d'une composition pour l'utilisation selon l'une des revendications 1 à 7 et 9 à 10, laquelle méthode comprend les étapes suivantes consistant à :
1. préparer une solution aqueuse ou une suspension d'au moins un matériau porteur,
2. mélanger avec du sulfate de cholestéryle et, en cas échéant, un ou plusieurs agents actifs et/ou des substances auxiliaires,
3. verser le mélange de l'étape 2, et
4. sécher le mélange.

12. Méthode selon la revendication 11, dans laquelle dans l'étape 2, le sulfate de cholestéryle est mélangé en forme d'une solution vésicule aqueuse ou en forme de particules porteuses revêtues avec du sulfate de cholestéryle, en particulier des particules de gélatine revêtues avec du sulfate de cholestéryle.

13. Méthode selon l'une quelconque des revendications 11 à 12, dans laquelle l'étape 4 comprend de verser dans un moule, et l'étape 5 comprend de congeler le mélange dans le moule et de lyophiliser le mélange.

14. Méthode de préparation d'une composition pour l'utilisation selon l'une des revendications 1 à 6 et 8 à 10, laquelle méthode comprend d'appliquer le sulfate de cholestéryle sur un matériau porteur qui est en forme de couche, en particulier par revêtement par pulvérisation d'un matériau porteur qui est en forme de couche ou par laisser tremper le matériau porteur dans une solution contenant du sulfate de cholestéryle et ensuite sécher.
